# EUROPEAN PATENT SPECIFICATION

(11) **EP 4 074 277 B1**
(45) Date of publication and mention of the grant of the patent: **30.04.2025**
(21) Application number: 22163478.5
(22) Date of filing: 22.03.2022
(51) Int. Cl.: A61B 34/20, A61B 1/00, A61B 90/00, G06T 7/00, G06T 7/13, G06T 7/73, G16H 30/40, A61B 17/00, A61B 34/10, A61B 34/30, A61B 1/313

(54) **MEDICAL SUPPORT APPARATUS**
MEDIZINISCHE STÜTZVORRICHTUNG
APPAREIL DE SUPPORT MÉDICAL

(30) Priority: 14.04.2021 US 202163174717 P; 23.02.2022 US 202217678244
(43) Date of publication of application: 19.10.2022
(73) Proprietor: Olympus Corporation, Hachioji-shi Tokyo 192-8507 (JP)
(72) Inventor: YAMAMOTO, Shota, Tokyo, 192-8507 (JP); OGAWA, Ryohei, 22045 Hamburg (DE); ENDERS, Borg, 22045 Hamburg (DE); SUPPA, Per, 22045 Hamburg (DE)
(74) Representative: Gunzelmann, Rainer

(56) References cited:
- WO-A1-2020/110278
- WO-A1-2021/006228
- WO-A2-2020/163845
- US-A1- 2019 069 957
- US-A1- 2020 268 472
- GUPTA VISHAL ET AL: "Safe laparoscopic cholecystectomy: Adoption of universal culture of safety in cholecystectomy", WORLD JOURNAL OF GASTROINTESTINAL SURGERY, vol. 11, no. 2, 27 February 2019 (2019-02-27), pages 62 - 84, XP055960015, ISSN: 1948-9366, Retrieved from the Internet <URL:https://www.researchgate.net/profile/Vishal-Gupta-22/publication/330618898_Safe_laparoscopic_cholecystectomy_Adoption_of_universal_culture_of_safety_in_cholecystectomy/links/5c7bb65292851c69504f9312/Safe-laparoscopic-cholecystectomy-Adoption-of-universal-culture-of-safety-in-cholecystectomy.pdf> DOI: 10.4240/wjgs.v11.i2.62
- TOKUYASU TATSUSHI ET AL: "Development of an artificial intelligence system using deep learning to indicate anatomical landmarks during laparoscopic cholecystectomy", SURGICAL ENDOSCOPY, vol. 35, no. 4, 18 April 2020 (2020-04-18), pages 1651 - 1658, XP037414277, ISSN: 0930-2794, DOI: 10.1007/S00464-020-07548-X
- MISCHINGER HANS-JÖRG ET AL: "The "critical view of safety (CVS)" cannot be applied-What to do? Strategies to avoid bile duct injuries", EUROPEAN SURGERY, SPRINGER VIENNA, VIENNA, vol. 53, no. 3, 10 September 2020 (2020-09-10), pages 99 - 105, XP037476050, ISSN: 1682-8631, [retrieved on 20200910], DOI: 10.1007/S10353-020-00660-1

## Description

The present disclosure relates to a medical support apparatus and a medical support method for supporting medical treatment such as laparoscopic surgery.

Still to this day, surgery relies heavily on the anatomical knowledge of physicians. This is even more so in the case of laparoscopic surgery. In laparoscopic surgery, doctors do not have tactile feedback to understand the structure of the inside of the patient's body and relies solely on visual information.

Laparoscopic cholecystectomy is surgery performed for the purpose of removing the gallbladder. Therefore, it is necessary to identify two structures, the cystic duct and the cystic artery, and sequentially excise those structures. Under anatomically difficult situations, doctors need to be careful not to misidentify anatomical structures. In particular, doctors need to be careful not to misidentify a common bile duct and a cystic duct. Laparoscopic cholecystectomy is one of surgical operations performed by inexperienced doctors after training, and there is a possibility that laparoscopic cholecystectomy is performed by doctors who still do not have sufficient required anatomical knowledge or experience with common anatomical variations.

To make up for the doctor's lack of anatomical knowledge and experience, it is conceivable to use a surgery support system to guide the doctor to a recommended treatment area. For example, a surgery support system has been proposed that supports a doctor's procedure by superimposing a virtual image of an excision surface on a laparoscopic image during laparoscopic surgery (see, for example, Patent Literature 1).

[Patent Literature 1] Japanese Patent Application Publication NO. 2005-278888
WO 2020/110278 A1 discloses a trained model that is trained to output a position and shape of an object in a training image based on training data. In an example of an image, the detected position and shape of the object are displayed and the objects are displayed in different shapes and positions based on a predetermined degree of importance corresponding to the object.

US 2019/069957 A1 discloses systems for performing surgery. In the system, no-contact zones could be visually represented on the image, or imposed on the surgeon through haptic feedback that prevents (e.g., make it hard or stop entirely) the instruments from going in the forbidden regions.

WO 2021/006228 A1 discloses a medical observation system capable of improving recognition accuracy of images and appropriately controlling the motion of an arm device based on images. The system is configured to determine a second operative field image based on the position of the scissors and the position of the tumor 156 in a wider first operative field image so as to eliminate the operation of manually operating the forward-oblique viewing endoscope by the doctor.

US 2020/268472 A1 discloses a method of analysis of videos of surgical procedures. The method includes providing decision support for surgical procedures may include outputting a recommendation to a user to undertake and/or to avoid a specific action. An interface for providing decision support may include, for example, a display, a speaker, a light, a haptic feedback component, and/or any other input and/or feedback mechanism. A recommendation may include a description of a current surgical situation, guidance, an indication of preemptive or corrective measures, an indication of alternative approaches, danger zone mapping, and/or any other information that might inform the surgeon relative to a surgical procedure.

As mentioned above, in laparoscopic cholecystectomy, further improvements to existing surgery support systems are needed to make up for the doctor's lack of anatomical knowledge and experience.

In this background, a purpose of the present disclosure is to provide a technology that allows the doctor to recognize the highly recommended area for treatments during surgery.

A medical support apparatus according to the present invention is set forth in claim 1. A medical support apparatus according to one embodiment of the present disclosure has one processor or more, and the processor is configured to: acquire a medical image; and based on the medical image, generate a guidance display that indicates a boundary between segments whose recommendation levels for medical treatment are different in the medical image and that is to be superimposed on the medical image.

A medical support method according to another embodiment of the present disclosure, but not forming part of the claimed invention, includes: acquiring a medical image; and based on the medical image, generating a guidance display that indicates a boundary between segments whose recommendation levels for medical treatment are different in the medical image and that is to be superimposed on the medical image.

Optional combinations of the aforementioned constituting elements and implementations of the present disclosure in the form of methods, apparatuses, systems, recording mediums, and computer programs may also be practiced as additional modes of the present disclosure.
FIG. 1 is a diagram showing the configuration of a medical support system according to an embodiment;
FIG. 2 is a diagram that schematically shows the overall flow of laparoscopic cholecystectomy;
FIG. 3 is a diagram for explaining a method of generating a machine learning model according to the embodiment;
FIG. 4 is a diagram showing an example of a laparoscopic image in which B-SAFE landmarks are shown;
FIG. 5 is a diagram showing an example of a laparoscopic image in which a Rouviere's sulcus and a hilar plate are shown;
FIG. 6 is a flowchart showing the basic operation of a medical support apparatus according to the embodiment;
FIG. 7 is a diagram showing an example of an image in which a line graphic is superimposed on a laparoscopic image captured during laparoscopic cholecystectomy;
FIG. 8 is a diagram showing an example of an image in which an unsafe zone graphic is superimposed on a laparoscopic image captured during laparoscopic cholecystectomy;
FIG. 9 is a diagram showing an example of an image in which a safe zone graphic is superimposed on a laparoscopic image captured during laparoscopic cholecystectomy;
FIG. 10 is a diagram showing another example of an image in which a guidance display is superimposed on a laparoscopic image captured during laparoscopic cholecystectomy.
FIG. 11 is a diagram showing an example of four detection targets in a laparoscopic image captured during laparoscopic cholecystectomy;
FIG. 12 is a diagram showing a laparoscopic image in which the laparoscopic image shown in FIG. 11 is zoomed in;
FIG. 13 is a diagram showing an example of change in a laparoscopic image caused due to displacement of a laparoscope;
FIGS. 14A to 14B are diagrams showing an example of an image in which an excision area indicating the position of a cystic duct to be excised is superimposed on a laparoscopic image captured during laparoscopic cholecystectomy;
FIG. 15 is a diagram showing an example of an image in which three line graphics are superimposed on a laparoscopic image captured during laparoscopic cholecystectomy;
FIG. 16 is a diagram showing another example of an image in which three line graphics are superimposed on a laparoscopic image captured during laparoscopic cholecystectomy; and
FIG. 17 is a diagram showing an example of an image in which an animation graphic, in accordance with the invention, is superimposed on a laparoscopic image captured during laparoscopic cholecystectomy.

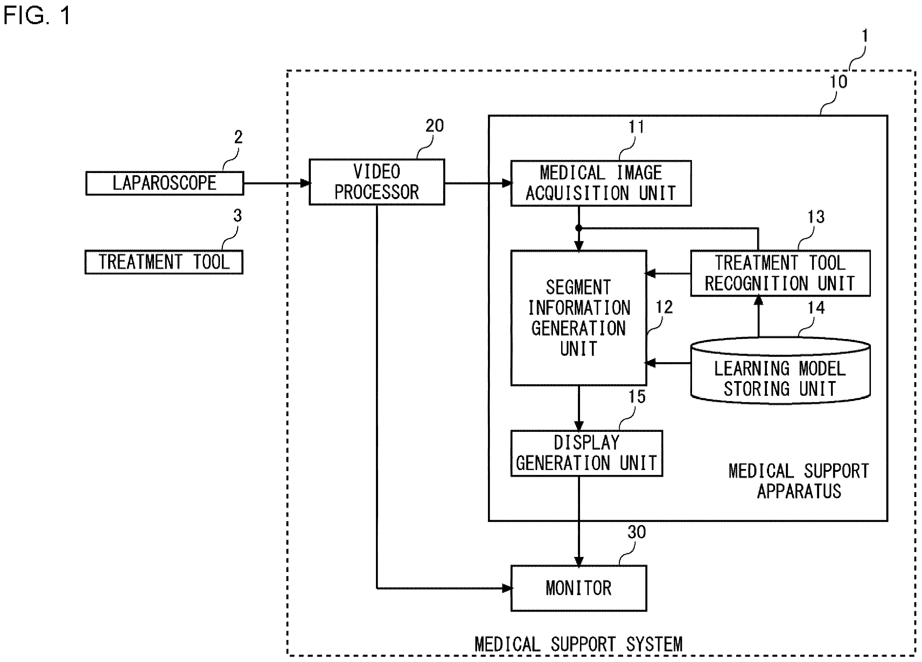

FIG. 1 shows the configuration of a medical support system 1 according to an embodiment. The medical support system 1 is a system for supporting laparoscopic surgery used in a surgical department. In laparoscopic surgery, an endoscope for abdominal surgery (hereinafter referred to as laparoscope 2) and a treatment tool 3 are inserted through several holes made in the patient's abdomen. The laparoscope 2 is not a flexible mirror used for examination of the stomach or large intestine and is formed using a hard metal mirror. As the treatment tool 3, forceps, a trocar, an energy device, or the like is used.

The medical support system 1 is provided in an operating room and includes a medical support apparatus 10, a video processor 20, and a monitor 30. The medical support apparatus 10 includes a medical image acquisition unit 11, a segment information generation unit 12, a treatment tool recognition unit 13, a learning model storing unit 14, and a display generation unit 15.

The configuration of the medical support apparatus 10 is implemented by hardware such as an arbitrary processor (for example, CPU and GPU), memory, auxiliary storage (for example, HDD and SSD), or other LSIs and by software such as a program or the like loaded into the memory. The figure depicts functional blocks implemented by the cooperation of hardware and software. Thus, a person skilled in the art should appreciate that there are many ways of accomplishing these functional blocks in various forms in accordance with the components of hardware only, software only, or the combination of both.

The laparoscope 2 has a light guide for illuminating the inside of the patient body by transmitting illumination light supplied from a light source device, and the distal end of the laparoscope 2 is provided with an illumination window for emitting the illumination light transmitted by the light guide to a subject and an imaging unit for imaging the subject at a predetermined cycle and outputting an imaging signal to the video processor 20. The imaging unit includes a solid-state imaging device (for example, a CCD image sensor or a CMOS image sensor) that converts incident light into an electric signal.

The video processor 20 performs image processing on the imaging signal photoelectrically converted by a solid-state imaging device of the laparoscope 2 so as to generate a laparoscopic image. The video processor 20 can also perform effect processing such as highlighting in addition to normal image processing such as A/D conversion and noise removal.

A laparoscopic image generated by the video processor 20 is output to the medical support apparatus 10 and is displayed on the monitor 30 after a guidance display is superimposed on the laparoscopic image by the medical support apparatus 10. As a failback function, the medical support apparatus 10 can be bypassed, and the laparoscopic image can be output directly from the video processor 20 to the monitor 30. Further, although FIG. 1 shows a configuration in which the medical support apparatus 10 and the video processor 20 are separated into separate devices, the medical support apparatus 10 and the video processor 20 may be integrated into one device.

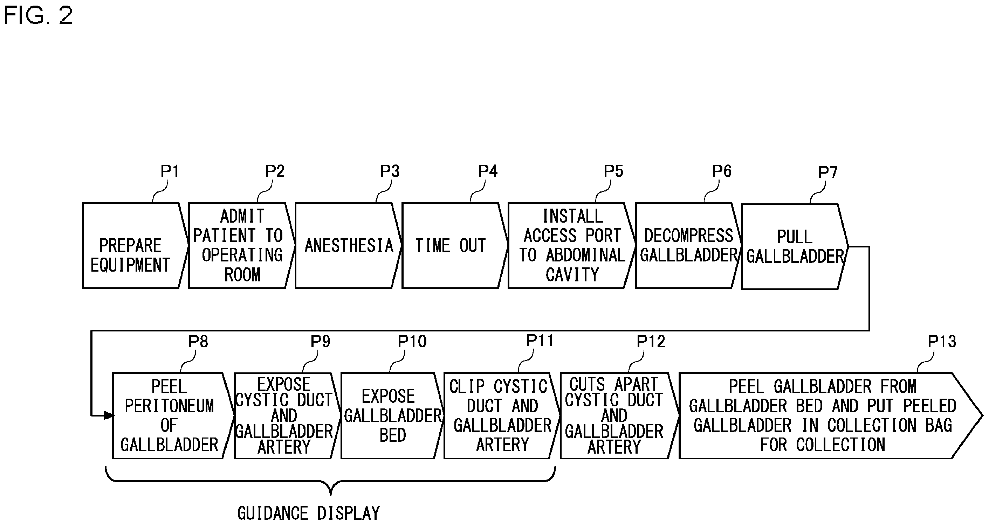

FIG. 2 is a diagram that schematically shows the overall flow of laparoscopic cholecystectomy. First, the equipment is prepared in the operating room (P1). In this step, the medical support system 1 is activated. Next, the patient is admitted to the operating room (P2). The anesthesiologist then administers general anesthesia to the patient (P3). Then, the entire surgical team conducts a time out (P4). More specifically, the team checks the patient, surgical site, procedure, etc. The surgeon then installs an access port to the abdominal cavity (P5). More specifically, a small hole is made in the abdomen and a trocar is inserted so as to install the access port. A laparoscope, forceps, and an energy device are inserted into the abdominal cavity through the trocar. Carbon dioxide is sent into the abdominal cavity from an insufflation device such that the abdominal cavity is filled with gas so as to secure a surgical work space.

Next, a surgeon decompresses the inside of the gallbladder (P6) and pulls the gallbladder with grasping forceps (P7). The surgeon then peels the peritoneum covering the gallbladder with a dissector (P8). The surgeon then exposes the cystic duct and gallbladder artery (P9) and exposes the gallbladder bed (P10). The surgeon then clips the cystic duct and gallbladder artery (P11) and cuts apart the cystic duct and gallbladder artery using scissors forceps (P12). Finally, the surgeon peels the gallbladder from the gallbladder bed with the dissector and puts the peeled gallbladder in a collection bag for collection.

The medical support apparatus 10 according to the embodiment assists the surgeon during the procedure to confirm the margin of safety by superimposing a guidance display on a laparoscopic image displayed in real time on the monitor 30 in steps P8 to P11.

The medical support system 1 according to the embodiment uses a machine learning model trained to recognize important anatomical landmarks from live images during the procedure.

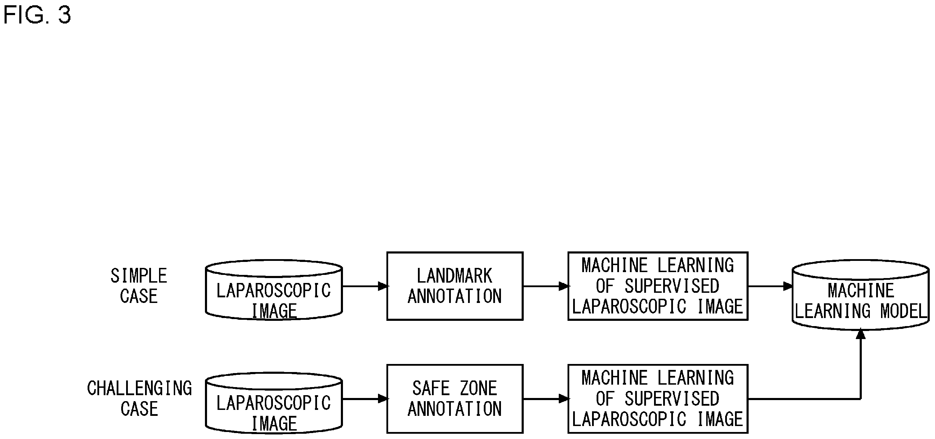

FIG. 3 is a diagram for explaining a method of generating a machine learning model according to the embodiment. In a simple case, an annotator with specialized knowledge such as a doctor annotates anatomical landmarks shown in numerous laparoscopic images. An AI/machine learning system machine-learns a supervised dataset of laparoscopic images with annotated anatomical landmarks as training data so as to generate a machine learning model for landmark detection. For example, CNN, RNN, LSTM, etc., which are a kind of deep learning, can be used as machine learning.

In a challenging case, a machine learning model for detecting safe zones from laparoscopic images of complex cases that do not show anatomical landmarks is generated. An annotator with a high degree of specialized knowledge, such as a skilled doctor, annotate safe zones in numerous laparoscopic images that do not show anatomical landmarks. For example, the annotator draws safe zone lines in the laparoscopic images. Further, annotations may be added to estimated positions of anatomical landmarks that are covered with adipose tissue or the like and are not shown in the laparoscopic images. An AI/machine learning system machine-learns a supervised dataset of laparoscopic images annotated with regard to safe zones as training data so as to generate a machine learning model for safe zone detection.

A machine learning model for detecting at least one of anatomical landmarks and safe zones generated as described above is registered in the learning model storing unit 14 of the medical support apparatus 10.

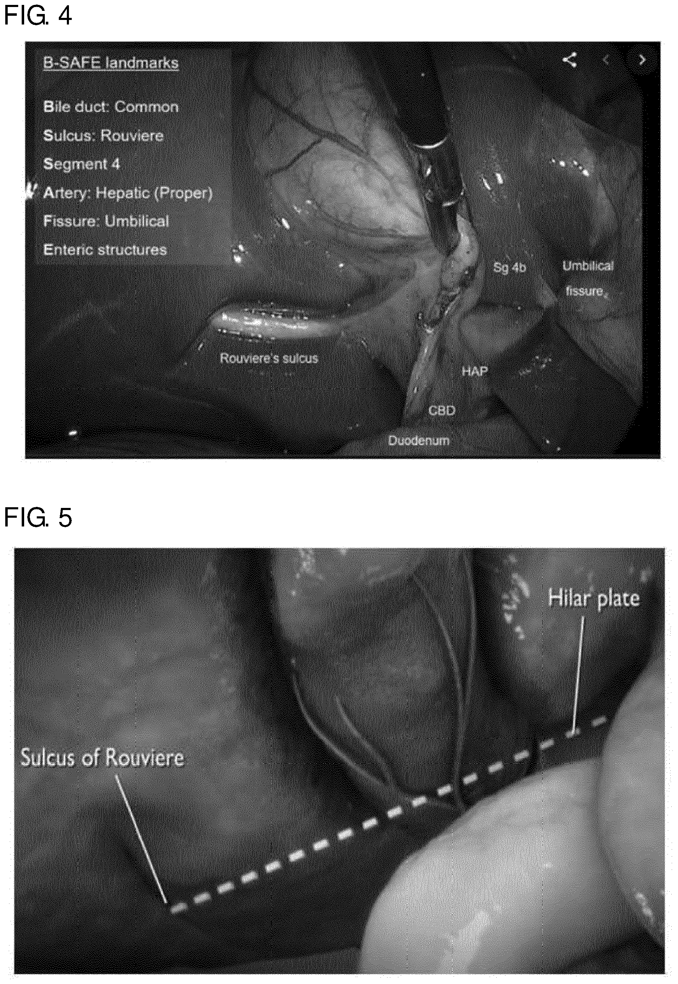

B-SAFE landmarks can be used as anatomical landmarks in laparoscopic images showing the gallbladder or laparoscopic images showing the surrounding tissue of the gallbladder captured during laparoscopic cholecystectomy. As for B-SAFE landmarks, a bile duct (B), a Rouviere's sulcus (S), the lower edge of a liver S4 (S), a hepatic artery (A), an umbilical fissure (F), and an intestinal structure (duodenum) (E) are used as the landmarks. In addition, a hilar plate can be also used as an anatomical landmark. FIG. 4 is a diagram showing an example of a laparoscopic image in which B-SAFE landmarks are shown. FIG. 5 is a diagram showing an example of a laparoscopic image in which a Rouviere's sulcus and a hilar plate are shown. The laparoscopic image showing the tissue around the gallbladder means a laparoscopic image showing at least one of any of the above-mentioned B-SAFE landmarks, the cystic duct, and the gallbladder artery.

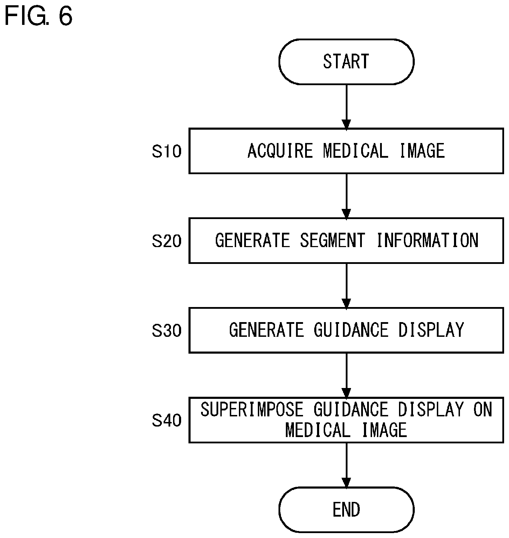

FIG. 6 is a flowchart showing the basic operation of the medical support apparatus 10 according to the embodiment. The medical image acquisition unit 11 acquires a medical image (in the present embodiment, a laparoscopic image showing the gallbladder or a laparoscopic image showing the tissue around the gallbladder) from the video processor 20 (S10). The segment information generation unit 12 generates segment information that regionally defines the level of safety of medical treatment (laparoscopic cholecystectomy in the present embodiment) based on a medical image acquired by the medical image acquisition unit 11 (S20). The display generation unit 15 generates a guidance display to be superimposed on the medical image based on the segment information generated by the segment information generation unit 12 (S30). The display generation unit 15 superimposes the generated guidance display on the medical image that is input from the video processor 20 (S40). A detailed description is now given in the following.

The segment information generation unit 12 estimates the positions of two or more anatomical landmarks based on the medical image input from the video processor 20. The segment information generation unit 12 detects the positions of two or more anatomical landmarks from the input medical image by using a learning model read from the learning model storing unit 14. The segment information generation unit 12 generates segment information based on the positions of the two or more detected anatomical landmarks (specifically, the relative positions of the two or more anatomical landmarks). The segment information is information that defines in the medical image the safety level of a region shown in the medical image when performing the medical treatment.

The display generation unit 15 can generate a line graphic indicating a boundary between a plurality of segments defined by segment information as a guidance display that is superimposed on the medical image. The display generation unit 15 superimposes the generated line graphic on the medical image as an on screen display (OSD).

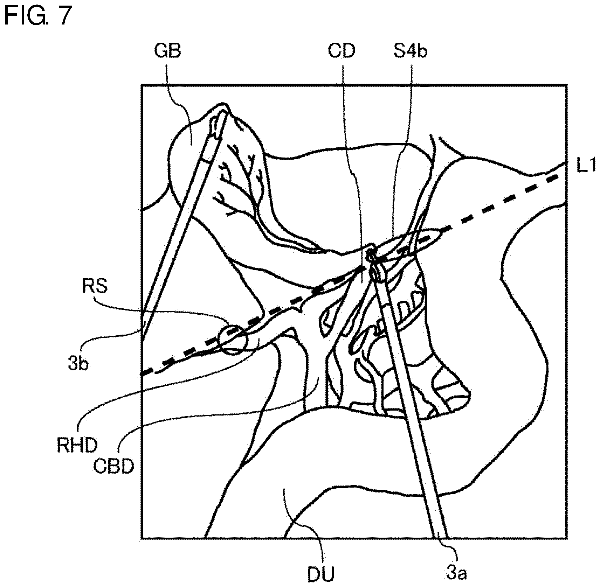

FIG. 7 is a diagram showing an example of an image in which a line graphic L1 is superimposed on a laparoscopic image captured during laparoscopic cholecystectomy. In the example shown in FIG. 7, the segment information generation unit 12 detects a Rouviere's sulcus RS and the lower edge S4b of a liver S4 as anatomical landmarks from the laparoscopic image showing a gallbladder GB. In addition, instead of the lower edge S4b of the liver S4, a hilar plate or an umbilical fissure located near the lower edge S4b of the liver S4 may be used.

In laparoscopic cholecystectomy, it is necessary to excise a cystic duct CD that connects to a gallbladder GB at a position before the cystic duct CD merges with the common hepatic duct that connects to the liver (the hepatic duct after a right hepatic duct RHD and a left hepatic duct merge with each other). If a common bile duct CBD after the merger of the cystic duct CD and the common hepatic duct is accidentally excised, bile cannot flow from the liver to a duodenum DU.

The segment information generation unit 12 generates a line passing through the Rouviere's sulcus RS and the lower edge S4b of the liver S4 that have been detected (hereinafter, referred to as an R4U line as required) and sets the segment above the R4U line as a safe zone segment and the segment below the R4U line as an unsafe zone segment. The display generation unit 15 generates a line graphic L1 showing a boundary (R4U line) between the safe zone segment and the unsafe zone segment and superimposes the line graphic L1 on the laparoscopic image.

The display generation unit 15 can generate a plate graphic showing the region of one segment defined by the segment information as a guidance display that is superimposed on the medical image. The display generation unit 15 superimposes the generated plate graphic on the medical image as an OSD.

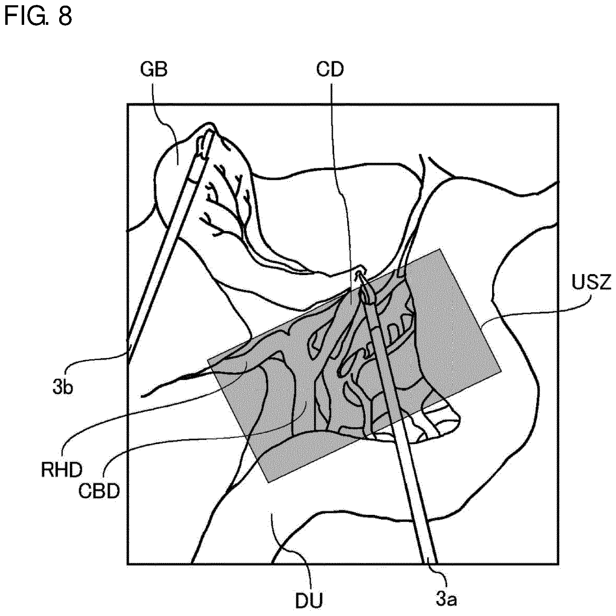

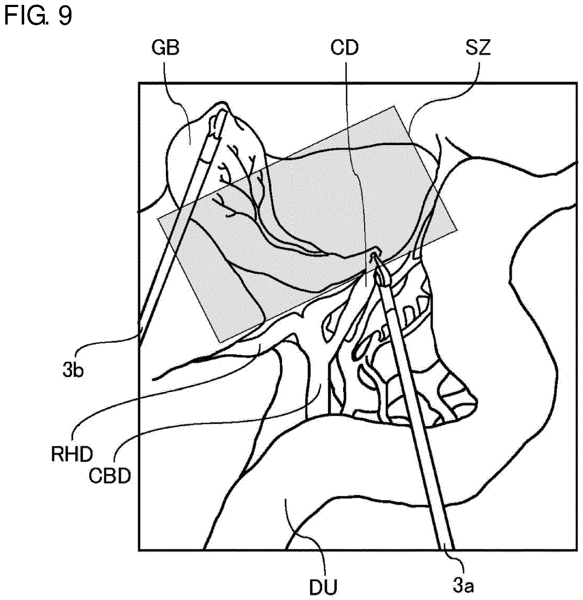

FIG. 8 is a diagram showing an example of an image in which an unsafe zone graphic USZ is superimposed on a laparoscopic image captured during laparoscopic cholecystectomy. FIG. 9 is a diagram showing an example of an image in which a safe zone graphic SZ is superimposed on a laparoscopic image captured during laparoscopic cholecystectomy.

In the example shown in FIG. 8, the display generation unit 15 generates the unsafe zone graphic USZ in the segment below the R4U line and superimposes the unsafe zone graphic USZ on a laparoscopic image. Both the unsafe zone graphic USZ and the line graphic L1 may be superimposed on the laparoscopic image. The unsafe zone graphic USZ is desirably generated in a color (e.g., red) that draws the attention of the doctor. In FIG. 8, the unsafe zone graphic USZ is formed by a rectangular plate. However, as long as the upper edge (upper side) of the plate lies along the R4U line, the unsafe zone graphic USZ may be formed by a parallelogram plate or a plate including a curved line. The size of the unsafe zone graphic USZ is set to a size that covers at least the common bile duct CBD.

In the example shown in FIG. 9, the display generation unit 15 generates the safe zone graphic SZ in the segment above the R4U line and superimposes the safe zone graphic SZ on a laparoscopic image. Both the safe zone graphic SZ and the line graphic L1 may be superimposed on the laparoscopic image. The safe zone graphic SZ is desirably generated in a color (e.g., green) that reminds the doctor that the safe zone graphic SZ is a recommended zone to be treated by the doctor. In FIG. 9, the safe zone graphic SZ is formed by a rectangular plate. However, as long as the lower edge (lower side) of the plate lies along the R4U line, the safe zone graphic SZ may be formed by a parallelogram plate or a plate including a curved line.

When displaying the unsafe zone graphic USZ and the safe zone graphic SZ in different colors, the display generation unit 15 may superimpose both the unsafe zone graphic USZ and the safe zone graphic SZ on the laparoscopic image.

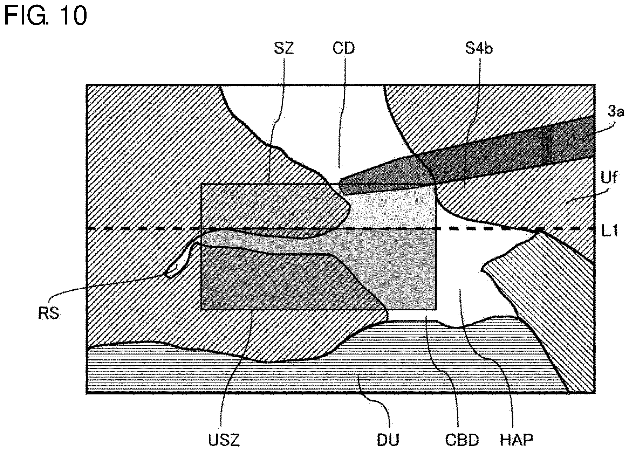

FIG. 10 is a diagram showing another example of an image in which a guidance display is superimposed on a laparoscopic image captured during laparoscopic cholecystectomy. The doctor performs a procedure for cholecystectomy in the region above the line graphic L1 showing the R4U line while looking at the laparoscopic image.

The treatment tool recognition unit 13 shown in FIG. 1 recognizes a treatment tool 3 in a medical image. The treatment tool recognition unit 13 detects the treatment tool 3 in a laparoscopic image by collating a laparoscopic image captured during the laparoscopic surgery with a template image of the treatment tool 3. As the template image, a plurality of images having different directions, projection lengths, and open/closed states are prepared for each treatment tool. Further, for an asymmetrical shape treatment tool whose shape on an image changes due to rotation, a plurality of images having different rotation angles are prepared.

The treatment tool recognition unit 13 generates an edge image in which the edge of the laparoscopic image is emphasized and detects the line segment shape from the edge image by using template matching, Hough transformation, or the like. The treatment tool recognition unit 13 collates the detected line segment shape with the template images and sets a treatment tool in a template image having the highest degree of matching as the detection result. The treatment tool 3 may be recognized by using a pattern detection algorithm using feature values such as scale-invariant feature transform (SIFT) and speeded up robust features (SURF). Further, the treatment tool 3 may be recognized based on a learning model in which the position (edge, display region) of the treatment tool is annotated and machine-learned.

The display generation unit 15 generates a different guidance display according to the relative positional relationship between the treatment tool 3 recognized by the treatment tool recognition unit 13 and a segment defined by the segment information. The display generation unit 15 generates an alerting guidance display according to the relative positional relationship between the recognized treatment tool 3 and the unsafe zone segment.

For example, when a projecting portion of the distal end of the recognized treatment tool 3 falls within the unsafe zone segment, the display generating unit 15 generates an unsafe zone graphic USZ that is more emphasized (e.g., in darker red) as an alerting guidance display.

In the examples shown in FIGS. 7-9, scissors forceps 3a and grasping forceps 3b are detected in a laparoscopic image. When a projecting portion of the distal end of the scissor forceps 3a falls within the unsafe zone segment, the display generation unit 15 generates a more emphasized unsafe zone graphic USZ.

When the projecting portion of the distal end of the treatment tool 3 is located near the R4U line, the alert is turned on/off frequently. As a countermeasure, an R4U zone in which the R4U line is widened in the width direction may be provided, and the R4U zone may be used as a dead zone. The display generation unit 15 stops the on/off of the alert while the projecting portion of the distal end of the treatment tool 3 falls within the R4U zone. The display generation unit 15 turns off the alert when the projecting portion of the distal end of the treatment tool 3 moves outside of the R4U zone and into the safe zone segment and turns on the alert when the projecting portion moves into the unsafe zone segment.

The alert display based on the recognition of the treatment tool 3 is not an essential function and can be omitted. In that case, the treatment tool recognition unit 13 of the medical support apparatus 10 can be omitted. Further, the display generation unit 15 does not need to superimpose a guidance display on a laparoscopic image during the normal state and may superimpose an alerting guidance display during an alert state or when the state is predicted to become the alert state. In this case, a guidance display is displayed only when the treatment tool 3 approaches the unsafe zone.

The segment information generation unit 12 can extract two or more feature points from the medical image in addition to the above two or more anatomical landmarks. The segment information generation unit 12 detects feature values such as SIFT and SURF and extracts feature points from the medical image. The feature points may be points that can be tracked by the segment information generation unit 12, and points or the like are used that have a scar peculiar to the patient or have a special color.

The segment information generation unit 12 generates segment information based on the positions of the two or more anatomical landmarks and the positions of the two or more feature points. The segment information generation unit 12 defines a specific segment based on the relative positional relationship of these four or more detection targets.

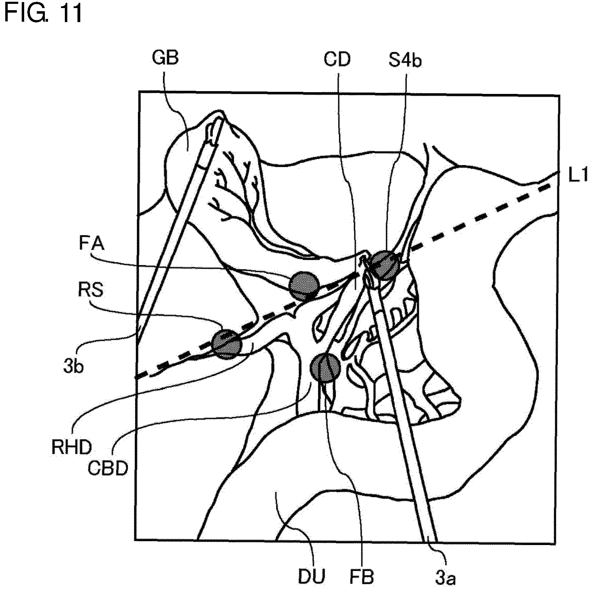

FIG. 11 is a diagram showing an example of four detection targets in a laparoscopic image captured during laparoscopic cholecystectomy. The four detection targets are the Rouviere's sulcus RS, the lower edge S4b of the liver S4, a first feature point FA, and a second feature point FB.

When the detection of at least one anatomical landmark is interrupted, the segment information generation unit 12 generates segment information obtained after the detection of at least one anatomical landmark is interrupted based on the positions of at least the two or more feature points. The display generation unit 15 changes the guidance display to be superimposed on the medical image based on the segment information obtained after the detection of at least one anatomical landmark is interrupted. That is, even if at least one of the anatomical landmarks is out of the field of view of the laparoscope 2 due to zooming in or moving of the laparoscope 2, the segment information generation unit 12 can estimate the relative positional relationship between two or more anatomical landmarks based on the positions of at least the two or more feature points.

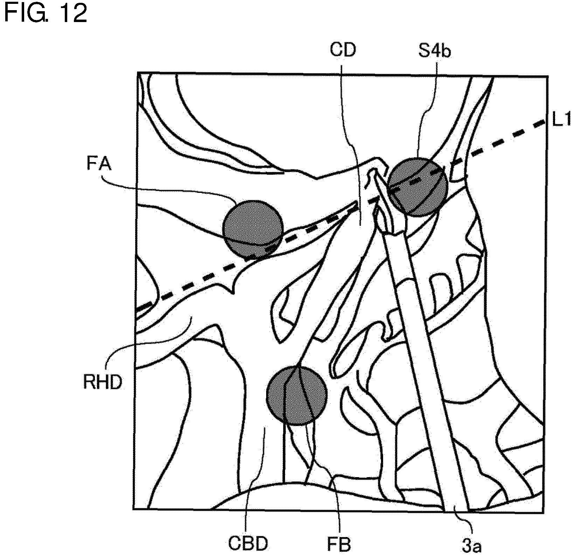

FIG. 12 is a diagram showing a laparoscopic image in which the laparoscopic image shown in FIG. 11 is zoomed in. In the laparoscopic image shown in FIG. 12, the Rouviere's sulcus RS, which is one of the anatomical landmarks, is out of the field of view of the laparoscope 2. Even in this case, the segment information generation unit 12 can generate the R4U line based on the relative positional relationship among the lower edge S4b of the liver S4, the first feature point FA, and the second feature point FB.

When the field of view of the laparoscope 2 changes, the segment information generation unit 12 tracks, instead of the relative positional relationship of at least the two or more feature points, the movement of the two or more feature points so as to generate a R4U line. In that case, the segment information generation unit 12 tracks the positions of two or more feature points extracted from the medical image by using the Kanade-Lucas-Tomasi feature tracker (KLT) method, the Mean-Shift search, or the like.

When the detection of at least one anatomical landmark is interrupted, the segment information generation unit 12 estimates positional change of a segment defined by the segment information obtained after the detection of at least one anatomical landmark is interrupted based on positional change of at least the two or more feature points. The display generation unit 15 changes the position of the guidance display to be superimposed on the medical image based on the positional change of the segment obtained after the detection of at least one anatomical landmark is interrupted.

When a laparoscopic surgery support robot is used, the medical support apparatus 10 can acquire positional change information (hereinafter, referred to as motion information) of the laparoscope 2 attached to the robot arm based on the amount of movement of each drive joint of the robot arm. In this case, the segment information generation unit 12 acquires the motion information of the laparoscope 2 from the robot system when the detection of at least one anatomical landmark is interrupted. Based on the acquired motion information of the laparoscope 2, the segment information generation unit 12 estimates positional change of the segment defined by the segment information obtained after the detection of at least one anatomical landmark is interrupted. The display generation unit 15 changes the position of the guidance display to be superimposed on the medical image based on the positional change of the segment obtained after the detection of at least one anatomical landmark is interrupted.

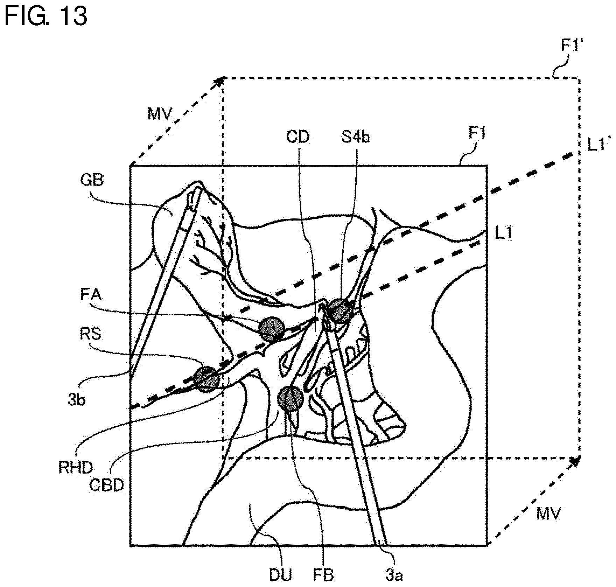

FIG. 13 is a diagram showing an example of change in a laparoscopic image caused due to displacement of the laparoscope 2. A field of view F1 of the laparoscope 2 changes to a field of view F1' due to the displacement of the laparoscope 2 by the robot arm. In the field of view F1', the Rouviere's sulcus RS, which is one of the anatomical landmarks, is not visible. The segment information generation unit 12 identifies the positional change of the R4U line based on motion information MV of the laparoscope 2. Thereby, the display generation unit 15 allows the line graphic L1 that is based on the R4U line to follow the displacement of the laparoscope 2.

When the laparoscopic surgery support robot is not used, the movement of the laparoscope 2 is detected by tracking a specific marker in the laparoscopic image by image processing. The segment information generation unit 12 can also use this motion information of the laparoscope 2 detected by the image processing to estimate the displacement of the segment in the laparoscopic image. More accurate movement and operation information of the laparoscope 2 can be acquired when a laparoscopic surgery support robot is used. Thus, the position of an anatomical landmark outside the field of view can be estimated more accurately.

When a plurality of feature points are detected in the laparoscopic image, the segment information generation unit 12 desirably selects a feature point at a position along the R4U line or a position close to the R4U line. If two feature points can be detected inside two anatomical landmarks along the R4U line, the R4U line can be easily generated even when the anatomical landmarks disappear from the field of view of the laparoscope 2 due to a zoom-in operation performed by the doctor or the like. Further, by tracking the movement of the laparoscope 2, the accuracy of estimating the R4U line can be improved. The positions of anatomical landmarks (e.g., Rouviere's sulcus RS and lower edge S4b of liver S4) outside the field of view can also be easily estimated.

The segment information generation unit 12 can track the movement of tissue shown in the medical image.

The tissue may be a tissue to be excised (e.g., cystic duct). When the tissue to be excised is learned by a learning model, the segment information generation unit 12 uses the learning model so as to detect the tissue to be excised. When the tissue to be excised is registered as another dictionary data, the segment information generation unit 12 uses the dictionary data so as to detect the tissue to be excised.

The segment information generation unit 12 can track the detected tissue to be excised by a motion tracking function. The display generation unit 15 generates a guidance display that follows the movement of the tissue based on the tracking result of the movement of the tissue.

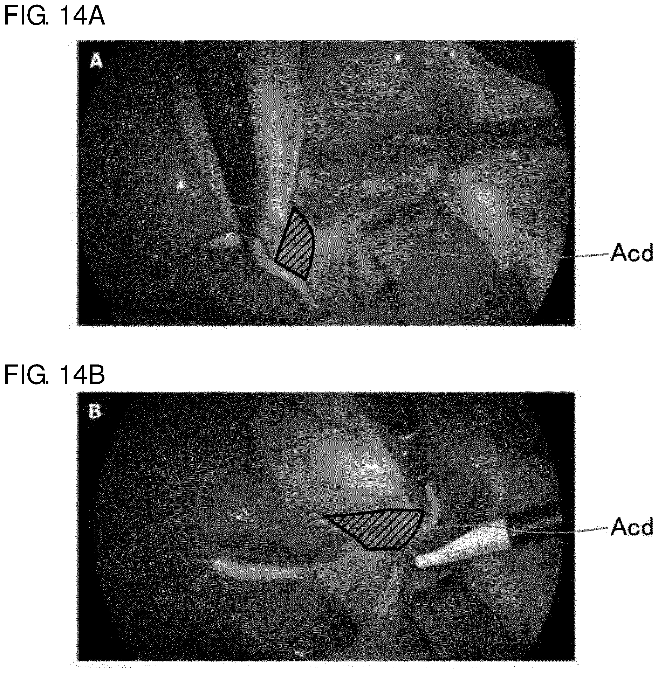

FIGS. 14A to 14B are diagrams showing an example of an image in which an excision area Acd indicating the position of a cystic duct CD to be excised is superimposed on a laparoscopic image captured during laparoscopic cholecystectomy. The display generation unit 15 changes the position of the excision area Acd indicating the position of the cystic duct CD according to the movement of the cystic duct CD to be excised in the laparoscopic image. The excision area Acd is superimposed on the laparoscopic image, for example, with a green marker.

The segment information generation unit 12 can also track the movement of two or more detected anatomical landmarks by the motion tracking function. Although not shown in FIGS. 14A to 14B, based on the tracking result of the movement of the two or more anatomical landmarks, the display generation unit 15 can change a guidance display (e.g., line graphic L1) that is based on the two or more anatomical landmarks.

The display generation unit 15 can generate a plurality of line graphics indicating a boundary between a plurality of segments defined by segment information in stages as a guidance display that is superimposed on the medical image. The display generation unit 15 superimposes the plurality of generated line graphics on the medical image. The plurality of line graphics may be three or more line graphics each passing through two anatomical landmarks and being at positions different from one another. The three or more line graphics are desirably generated in colors different from one another.

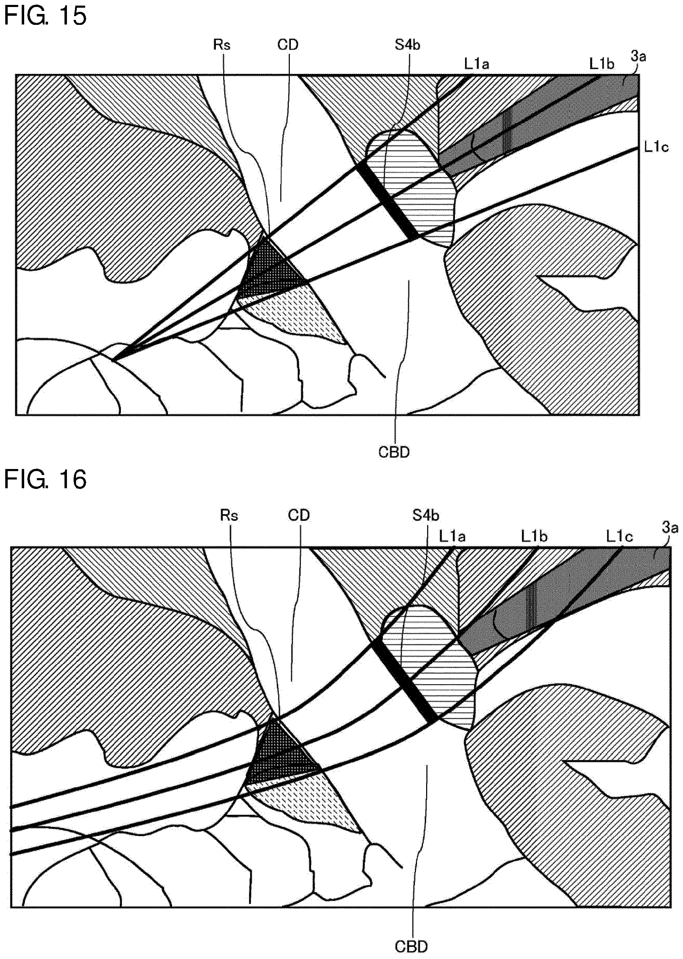

FIG. 15 is a diagram showing an example of an image in which three line graphics L1a to L1c are superimposed on a laparoscopic image captured during laparoscopic cholecystectomy. In the example shown in FIG. 15, the segment information generation unit 12 detects a Rouviere's sulcus RS and the lower edge S4b of a liver S4 as anatomical landmarks. The display generation unit 15 superimposes color tiles of different colors on the Rouviere's sulcus RS and the lower edge S4b of the liver S4.

In the example shown in FIG. 15, on the laparoscopic image, the display generation unit 15 superimposes a green first line graphic L1a passing through the upper end of the Rouviere's sulcus RS and the upper end of the lower edge S4b of the liver S4, a yellow third line graphic L1c passing through the lower end of the Rouviere's sulcus RS and the lower end of the lower edge S4b of the liver S4, and a yellowish green second line graphic L1b passing midway between the first line graphic L1a and the third line graphic L1c.

FIG. 16 is a diagram showing another example of an image in which three line graphics L1a to L1c are superimposed on a laparoscopic image captured during laparoscopic cholecystectomy. Although FIG. 15 shows an example in which the three line graphics L1a to L1c are formed by a straight line, the three line graphics L1a to L1c may be formed by a curved line as shown in FIG. 16.

In the example shown in FIGS. 15 to 16, the segment above the first line graphic L1a is the first segment (safe zone segment), and the segment below the third line graphic L1c is the second segment (unsafe zone segment) in which the safety is lower than that in the first segment. The three line graphics L1a to L1c divide the first segment and the second segment in stages.

The display generation unit 15 can generate an animation graphic in which a plurality of line graphics move from the second segment to the first segment as a guidance display in the boundary area between the first segment and the second segment.

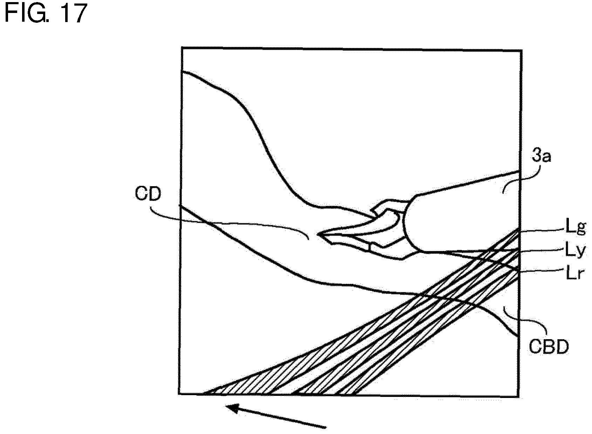

FIG. 17 is a diagram showing an example of an image in which an animation graphic is superimposed on a laparoscopic image captured during laparoscopic cholecystectomy. Although not shown in FIG. 17, the segment information generation unit 12 detects the Rouviere's sulcus RS and the umbilical fissure as anatomical landmarks.

In the example shown in FIG. 17, in the boundary area between the first segment and the second segment, the display generation unit 15 superimposes three line graphics: a red line graphic Lr; a yellow line graphic Ly; and a green line graphic Lg, on the laparoscopic image in this order from the bottom. The three line graphics move from the bottom end of the boundary area to the top end. More specifically, the red line graphic Lr springs up from the lower end of the boundary area, changes from red to yellow to green as the line graphic moves toward the upper end of the boundary area, and disappears when the line graphic reaches the upper end of the boundary area. The number of line graphics displayed in the boundary area may be four or more.

By displaying an animation graphic in which a plurality of line graphics move like waves from the unsafe zone to the safe zone in the boundary area, the doctor can be visually guided to move away from the unsafe zone and toward the safe zone.

In the above-described embodiment, when the segment information generation unit 12 cannot detect the R4U line, the display generation unit 15 may display an alert message on the monitor 30 to alert the doctor who is performing the procedure. In response to this alert message, an inexperienced doctor can consult with a skilled doctor or hand over the procedure to a skilled doctor, which contributes to minimizing the risk of damage to the common bile duct.

As explained above, according to the present embodiment, the doctor can fully recognize which parts are safer and which parts are less safe during laparoscopic surgery. The doctor can safely perform laparoscopic surgery by performing the procedure on the first segment. Superimposing the various guidance displays described above on a laparoscopic image can prevent accidental excision of the common bile duct in laparoscopic cholecystectomy and encourage the excision of the cystic duct and gallbladder artery at appropriate positions. For example, by displaying an R4U line as a guidance display, even an inexperienced doctor can always be aware of the R4U line and perform the procedure above the R4U line.

Described above is an explanation on the present disclosure based on the embodiments. These embodiments are intended to be illustrative only, and it will be obvious to those skilled in the art that various modifications to constituting elements and processes could be developed and that such modifications are also within the scope of the present disclosure.

In the above-mentioned embodiment, anatomical landmarks are detected from a captured laparoscopic image using a machine learning model. In this regard, feature values such SIFT, SURF, edges, and corners may be detected from a captured laparoscopic image, and anatomical landmarks may be detected based on the feature description of the laparoscopic image.

If a machine learning model for safe zone detection is prepared as in the challenge case described above, segment information can be directly generated without detecting anatomical landmarks. In this case, a safe zone and an unsafe zone in the laparoscopic image can be identified even when anatomical landmarks are not shown in the laparoscopic image.

## Claims

1. A medical support apparatus (10) comprising:
one processor (11, 12, 15) or more, wherein
the processor (11, 12, 15) is configured to:
acquire a laparoscopic image captured during laparoscopic cholecystectomy; and
based on the laparoscopic image, generate a guidance display that includes a plurality of lines indicating in stages a boundary between segments whose recommendation levels for medical treatment are different in the laparoscopic image and that is to be superimposed on the laparoscopic image, wherein
the processor (11, 12, 15) is configured to generate an animation where, in a boundary area between a first segment and a second segment whose recommendation level for medical treatment is lower than that of the first segment, the plurality of lines move from the second segment to the first segment as the guidance display.

2. The medical support apparatus (10) according to claim 1, wherein
the processor (11, 12, 15) is configured to:
estimate the positions of two or more anatomical landmarks based on the laparoscopic image; and
based on the estimated positions of the two or more anatomical landmarks, generate the guidance display.

3. The medical support apparatus (10) according to claim 2, wherein
the processor (11, 12, 15) is configured to:
acquire the laparoscopic image showing a gallbladder; and
the two or more anatomical landmarks include at least one of a Ruviere's sulcus, the lower edge of a liver S4, a hilar plate, a hepatic artery, and an umbilical fissure.

4. The medical support apparatus (10) according to claim 1, wherein
the processor (11, 12, 15) is configured to generate at least one of a line graphic indicating the boundary between segments and a plate that indicates the region of one segment and whose edge indicates the boundary between segments.

5. The medical support apparatus (10) according to claim 2, wherein
the processor (11, 12, 13, 15) is configured to generate the guidance display based on the relative positions of the two or more anatomical landmarks.

6. The medical support apparatus (10) according to claim 1, wherein
the processor (11, 12, 13, 15) is configured to:
recognize a treatment tool (3) in the laparoscopic image; and
change the presence/absence or display mode of the guidance display according to the relative positional relationship between the treatment tool (3) and the segments.

7. The medical support apparatus (10) according to claim 6, wherein
the processor (11, 12, 15) is configured to generate the guidance display, which serves as an alert, according to the relative positional relationship between the treatment tool (3) and the segments.

8. The medical support apparatus (10) according to claim 2, wherein
the processor (11, 12, 15) is configured to:
further extract two or more feature points that have a scar peculiar to a patient or have a special color from the laparoscopic image; and
generate the guidance display based on the positions of the two or more anatomical landmarks and the positions of the two or more feature points.

9. The medical support apparatus (10) according to claim 8, wherein
the processor (11, 12, 15) is configured to, when the detection of at least one of the anatomical landmarks is interrupted,
change the guidance display based on the positions of at least the two or more feature points obtained after the detection of at least one of the anatomical landmarks is interrupted.

10. The medical support apparatus (10) according to claim 2, wherein
the processor (11, 12, 15) is configured to:
extract two or more feature points from the laparoscopic image; track positional change of the two or more feature points; when the detection of at least one of the anatomical landmarks is interrupted, estimate positional change of the segments obtained after the detection of at least one of the anatomical landmarks is interrupted based on the positional change of the two or more feature points; and
change the guidance display based on the positional change of the segments obtained after the detection of at least one of the anatomical landmarks is interrupted.

11. The medical support apparatus (10) according to claim 2, wherein
the processor (11, 12, 15) is configured to:
acquire the laparoscopic image captured by an endoscope (2);
acquire motion information of the endoscope (2) when the detection of at least one of the anatomical landmarks is interrupted; and
change the position of the guidance display based on the motion information of the endoscope (2) obtained after the detection of at least one of the anatomical landmarks is interrupted.

12. The medical support apparatus (10) according to claim 1, wherein
the processor (11, 12, 15) is configured to:
track the movement of tissue shown in the laparoscopic image; and
generate a guidance display that follows the movement of the tissue based on the tracking result of the movement of the tissue.

13. The medical support apparatus (10) according to claim 1, wherein
the processor (11, 12, 15) is configured to display the laparoscopic image on which the guidance display is superimposed on a monitor (30) connected to the medical support apparatus (10).

14. The medical support apparatus (10) according to claim 4, wherein
the processor (11, 12, 15) is configured to:
acquire the laparoscopic image showing a gallbladder or tissue around the gallbladder; and
the line or an edge of the plate passes through a Rouviere's sulcus and the lower edge of a liver S4 shown in the laparoscopic image.

15. The medical support apparatus (10) according to claim 4, wherein
the processor (11, 12, 15) is configured to acquire the laparoscopic image showing a gallbladder or tissue around the gallbladder, and
the line or an edge of the plate passes through a Rouviere's sulcus and an umbilical fissure shown in the laparoscopic image.

16. The medical support apparatus (10) according to claim 1, wherein
the plurality of lines are three or more lines each passing through two anatomical landmarks and being at positions different from one another.

17. The medical support apparatus (10) according to claim 16, wherein
the three or more lines are in colors that are different from one another.

18. The medical support apparatus (10) according to claim 1, wherein
the laparoscopic image shows a gallbladder or tissue around the gallbladder.

## Patentansprüche

1. Eine medizinische Unterstützungsapparatur (10), umfassend:
einen oder mehrere Prozessoren (11, 12, 15), wobei
der Prozessor (11, 12, 15) dazu konfiguriert ist:
ein während einer laparoskopischen Cholezystektomie aufgenommenes laparoskopisches Bild zu erfassen; und
basierend auf dem laparoskopischen Bild eine Führungsanzeige zu erzeugen, die mehrere Linien umfasst, die stufenweise eine Grenze zwischen Segmenten angeben, deren Empfehlungsniveaus für die medizinische Behandlung im laparoskopischen Bild unterschiedlich sind und die auf dem laparoskopischen Bild zu überlagern ist, wobei
der Prozessor (11, 12, 15) dazu konfiguriert ist, eine Animation zu erzeugen, bei der sich in einem Grenzbereich zwischen einem ersten Segment und einem zweiten Segment, dessen Empfehlungsniveau für die medizinische Behandlung niedriger ist als das des ersten Segments, die mehreren Linien als die Führungsanzeige vom zweiten Segment zu dem ersten Segment bewegen.

2. Die medizinische Unterstützungsapparatur (10) nach Anspruch 1, wobei
der Prozessor (11, 12, 15) dazu konfiguriert ist:
die Positionen von zwei oder mehr anatomischen Landmarken basierend auf dem laparoskopischen Bild zu schätzen; und
basierend auf den geschätzten Positionen der zwei oder mehr anatomischen Landmarken die Führungsanzeige zu erzeugen.

3. Die medizinische Unterstützungsapparatur (10) nach Anspruch 2, wobei
der Prozessor (11, 12, 15) dazu konfiguriert ist:
das laparoskopische Bild, das eine Gallenblase zeigt, zu erfassen; und
die zwei oder mehr anatomischen Landmarken umfassen mindestens eine von: ein Ruviere's Sulcus, die untere Kante eines Leber-S4, eine Hilarplatte, eine Leberarterie und eine Nabelspalte.

4. Die medizinische Unterstützungsapparatur (10) nach Anspruch 1, wobei
der Prozessor (11, 12, 15) dazu konfiguriert ist, mindestens eine von Folgendem zu erzeugen: eine Liniengrafik, die die Grenze zwischen Segmenten angibt, und eine Fläche, die den Bereich eines Segments kennzeichnet und deren Rand die Grenze zwischen Segmenten anzeigt.

5. Die medizinische Unterstützungsapparatur (10) nach Anspruch 2, wobei
der Prozessor (11, 12, 13, 15) dazu konfiguriert ist, die Führungsanzeige basierend auf den relativen Positionen der zwei oder mehr anatomischen Landmarken zu erzeugen.

6. Die medizinische Unterstützungsapparatur (10) nach Anspruch 1, wobei
der Prozessor (11, 12, 13, 15) dazu konfiguriert ist:
ein Behandlungsinstrument (3) im laparoskopischen Bild zu erkennen; und
Vorhandensein/Fehlen oder Anzeigemodus der Führungsanzeige entsprechend der relativen positionalen Beziehung zwischen dem Behandlungsinstrument (3) und den Segmenten zu ändern.

7. Die medizinische Unterstützungsapparatur (10) nach Anspruch 6, wobei
der Prozessor (11, 12, 15) dazu konfiguriert ist, die Führungsanzeige, die als Warnhinweis dient, entsprechend der relativen positionalen Beziehung zwischen dem Behandlungsinstrument (3) und den Segmenten zu erzeugen.

8. Die medizinische Unterstützungsapparatur (10) nach Anspruch 2, wobei
der Prozessor (11, 12, 15) dazu konfiguriert ist:
ferner zwei oder mehr Merkmalspunkte, die eine für den Patienten eigentümliche Narbe aufweisen oder eine besondere Farbe haben, aus dem laparoskopischen Bild zu extrahieren; und
die Führungsanzeige basierend auf den Positionen der zwei oder mehr anatomischen Landmarken und den Positionen der zwei oder mehr Merkmalspunkte zu erzeugen.

9. Die medizinische Unterstützungsapparatur (10) nach Anspruch 8, wobei
der Prozessor (11, 12, 15) dazu konfiguriert ist, wenn die Detektion mindestens einer der anatomischen Landmarken unterbrochen wird,
die Führungsanzeige basierend auf den Positionen mindestens der zwei oder mehr Merkmalspunkte, die nach der Unterbrechung der Detektion mindestens einer der anatomischen Landmarken erhalten werden, zu ändern.

10. Die medizinische Unterstützungsapparatur (10) nach Anspruch 2, wobei
der Prozessor (11, 12, 15) dazu konfiguriert ist:
zwei oder mehr Merkmalspunkte aus dem laparoskopischen Bild zu extrahieren; eine Positionsänderung der zwei oder mehr Merkmalspunkte zu verfolgen; wenn die Detektion mindestens einer der anatomischen Landmarken unterbrochen wird, die Positionsänderung der Segmente, die nach der Unterbrechung der Detektion mindestens einer der anatomischen Landmarken erhalten wird, basierend auf der Positionsänderung der zwei oder mehr Merkmalspunkte zu schätzen; und
die Führungsanzeige basierend auf der Positionsänderung der Segmente, die nach der Unterbrechung der Detektion mindestens einer der anatomischen Landmarken erhalten wird, zu ändern.

11. Die medizinische Unterstützungsapparatur (10) nach Anspruch 2, wobei
der Prozessor (11, 12, 15) dazu konfiguriert ist:
das von einem Endoskop (2) aufgenommene laparoskopische Bild zu erfassen; Bewegungsinformationen des Endoskops (2) zu erfassen, wenn die Detektion mindestens einer der anatomischen Landmarken unterbrochen wird; und
die Position der Führungsanzeige basierend auf den Bewegungsinformationen des Endoskops (2), die nach der Unterbrechung der Detektion mindestens einer der anatomischen Landmarken erhalten werden, zu ändern.

12. Die medizinische Unterstützungsapparatur (10) nach Anspruch 1, wobei
der Prozessor (11, 12, 15) dazu konfiguriert ist:
die Bewegung von Gewebe, das im laparoskopischen Bild dargestellt ist, zu verfolgen; und
eine Führungsanzeige zu erzeugen, die der Gewebebewegung folgt, basierend auf dem Verfolgungsergebnis der Gewebebewegung.

13. Die medizinische Unterstützungsapparatur (10) nach Anspruch 1, wobei der Prozessor (11, 12, 15) dazu konfiguriert ist, das laparoskopische Bild, auf dem die Führungsanzeige überlagert ist, auf einem Monitor (30), der an die medizinische Unterstützungsapparatur (10) angeschlossen ist, anzuzeigen.

14. Die medizinische Unterstützungsapparatur (10) nach Anspruch 4, wobei
der Prozessor (11, 12, 15) dazu konfiguriert ist:
das laparoskopische Bild, das eine Gallenblase oder Gewebe um die Gallenblase herum zeigt, zu erfassen; und
die Linie oder ein Rand der Fläche verläuft durch ein Rouviere's Sulcus und die untere Kante eines Leber-S4, die im laparoskopischen Bild dargestellt sind.

15. Die medizinische Unterstützungsapparatur (10) nach Anspruch 4, wobei
der Prozessor (11, 12, 15) dazu konfiguriert ist, das laparoskopische Bild zu erfassen, das eine Gallenblase oder Gewebe um die Gallenblase herum zeigt, und
die Linie oder ein Rand der Fläche verläuft durch ein Rouviere's Sulcus und eine Nabelspalte, die im laparoskopischen Bild dargestellt sind.

16. Die medizinische Unterstützungsapparatur (10) nach Anspruch 1, wobei
die mehreren Linien drei oder mehr Linien sind, die jeweils durch zwei anatomische Landmarken verlaufen und sich an voneinander unterschiedlichen Positionen befinden.

17. Die medizinische Unterstützungsapparatur (10) nach Anspruch 16, wobei
die drei oder mehr Linien jeweils unterschiedliche Farben aufweisen.

18. Die medizinische Unterstützungsapparatur (10) nach Anspruch 1, wobei
das laparoskopische Bild eine Gallenblase oder Gewebe um die Gallenblase herum zeigt.

## Revendications

1. Appareil de support médical (10) comprenant:
un ou plusieurs processeurs (11, 12, 15), dans lequel
le processeur (11, 12, 15) est configuré pour:
acquérir une image par laparoscopie capturée au cours d'une cholécystectomie par laparoscopie; et
sur la base de l'image par laparoscopie, générer un affichage de guidage qui comprend une pluralité de lignes indiquant par étapes une limite entre les segments dont les niveaux de recommandation pour le traitement médical sont différents dans l'image par laparoscopie et qui doit être superposé à l'image par laparoscopie, dans lequel
le processeur (11, 12, 15) est configuré pour générer une animation dans laquelle, dans une zone limite entre un premier segment et un second segment dont le niveau de recommandation pour un traitement médical est inférieur à celui du premier segment, la pluralité de lignes se déplace du second segment au premier segment sous forme d'affichage de guidage.

2. Appareil de support médical (10) selon la revendication 1, dans lequel
le processeur (11, 12, 15) est configuré pour:
estimer les positions d'au moins deux repères anatomiques en fonction de l'image par laparoscopie; et
en fonction des positions estimées des au moins deux repères anatomiques, générer l'affichage de guidage.

3. Appareil de support médical (10) selon la revendication 2, dans lequel
le processeur (11, 12, 15) est configuré pour:
acquérir l'image par laparoscopie montrant une vésicule biliaire; et
les au moins deux repères anatomiques comprennent au moins l'un élément parmi le sulcus de Rouvière, le bord inférieur du foie S4, une plaque hilaire, une artère hépatique et une fissure ombilicale.

4. Appareil de support médical (10) selon la revendication 1, dans lequel
le processeur (11, 12, 15) est configuré pour générer au moins un graphique linéaire indiquant la limite entre les segments et une plaque indiquant la région d'un segment et dont le bord indique la limite entre les segments.

5. Appareil de support médical (10) selon la revendication 2, dans lequel
le processeur (11, 12, 13, 15) est configuré pour générer l'affichage de guidage sur la base des positions relatives des au moins deux repères anatomiques.

6. Appareil de support médical (10) selon la revendication 1, dans lequel
le processeur (11, 11, 12, 15) est configuré pour:
reconnaître un outil de traitement (3) dans l'image par laparoscopie; et
modifier la présence/l'absence ou le mode d'affichage de l'affichage de guidage en fonction de la relation de positionnement relatif entre l'outil de traitement (3) et les segments.

7. Appareil de support médical (10) selon la revendication 6, dans lequel
le processeur (11, 12, 15) est configuré pour générer l'affichage de guidage, qui sert d'alerte, en fonction de la relation de positionnement relatif entre l'outil de traitement (3) et les segments.

8. Appareil de support médical (10) selon la revendication 2, dans lequel
le processeur (11, 12, 15) est configuré pour:
extraire en outre au moins deux points caractéristiques qui présentent une cicatrice propre à un patient ou une couleur particulière de l'image par laparoscopie; et
générer l'affichage de guidage sur la base des positions des au moins deux repères anatomiques et des positions des au moins deux points caractéristiques.

9. Appareil de support médical (10) selon la revendication 8, dans lequel
le processeur (11, 12, 15) est configuré pour, lorsque la détection d'au moins un des repères anatomiques est interrompue,
modifier l'affichage de guidage en fonction des positions des au moins deux points caractéristiques obtenues après l'interruption de la détection d'au moins un des repères anatomiques.

10. Appareil de support médical (10) selon la revendication 2, dans lequel
le processeur (11, 12, 15) est configuré pour:
extraire au moins deux points caractéristiques de l'image par laparoscopie; suivre le changement de position des au moins deux points caractéristiques; lorsque la détection d'au moins un des repères anatomiques est interrompue, estimer le changement de position des segments obtenus après l'interruption de la détection d'au moins un des repères anatomiques sur la base du changement de position des au moins deux points caractéristiques; et
modifier l'affichage de guidage en fonction du changement de position des segments obtenu après l'interruption de la détection d'au moins un des repères anatomiques.

11. Appareil de support médical (10) selon la revendication 2, dans lequel
le processeur (11, 12, 15) est configuré pour:
acquérir l'image par laparoscopie capturée par un endoscope (2) ;
acquérir des informations sur les mouvements de l'endoscope (2) lorsque la détection d'au moins un des repères anatomiques est interrompue; et
modifier la position de l'affichage de guidage en fonction des informations sur les mouvements de l'endoscope (2) obtenues après l'interruption de la détection d'au moins un des repères anatomiques.

12. Appareil de support médical (10) selon la revendication 1, dans lequel
le processeur (11, 12, 15) est configuré pour:
suivre le mouvement du tissu représenté sur l'image par laparoscopie; et
générer un affichage de guidage qui suit le mouvement du tissu sur la base du résultat du suivi du mouvement du tissu.

13. Appareil de support médical (10) selon la revendication 1, dans lequel
le processeur (11, 12, 15) est configuré pour afficher l'image par laparoscopie sur laquelle l'affichage de guidage est superposé sur un moniteur (30) connecté à l'appareil de support médical (10).

14. Appareil de support médical (10) selon la revendication 4, dans lequel
le processeur (11, 12, 15) est configuré pour:
acquérir l'image par laparoscopie montrant une vésicule biliaire ou du tissu autour de la vésicule biliaire; et
la ligne ou un bord de la plaque passe par un sulcus de Rouvière et le bord inférieur d'un foie S4 représenté sur l'image par laparoscopie.

15. Appareil de support médical (10) selon la revendication 4, dans lequel
le processeur (11, 12, 15) est configuré pour acquérir l'image par laparoscopie montrant une vésicule biliaire ou le tissu autour de la vésicule biliaire; et
la ligne ou un bord de la plaque passe par un sulcus de Rouvière et une fissure ombilicale représenté sur l'image par laparoscopie.

16. Appareil de support médical (10) selon la revendication 1, dans lequel
la pluralité de lignes est constituée d'au moins trois lignes passant chacune par deux repères anatomiques et se trouvant à des positions différentes l'une de l'autre.

17. Appareil de support médical (10) selon la revendication 16, dans lequel
les trois lignes ou plus sont de couleurs différentes les unes des autres.

18. Appareil de support médical (10) selon la revendication 1, dans lequel
l'image par laparoscopie montre une vésicule biliaire ou le tissu autour de la vésicule biliaire.
